(19) Europäisches Patentamt
European Patent Office
Office européen des brevets

(11) Numéro de publication : **0 048 210 B1**

# (12) FASCICULE DE BREVET EUROPÉEN

(45) Date de publication du fascicule du brevet : **28.12.83**

(51) Int. Cl.³ : **C 07 C 51/56**, C 07 C 53/12

(21) Numéro de dépôt : **81420126.5**

(22) Date de dépôt : **24.08.81**

## (54) Procédé de préparation d'anhydrides d'acides carboxyliques par carbonylation.

(30) Priorité : **11.09.80 FR 8019875**

(43) Date de publication de la demande : **24.03.82 Bulletin 82/12**

(45) Mention de la délivrance du brevet : **28.12.83 Bulletin 83/52**

(84) Etats contractants désignés : **AT BE CH DE FR GB IT LI LU NL SE**

(56) Documents cités : **EP-A- 0 018 927**

(73) Titulaire : **RHONE-POULENC CHIMIE DE BASE
25, quai Paul Doumer
F-92408 Courbevoie (FR)**

(72) Inventeur : **Gauthier-Lafaye, Jean
21, rue Duguesclin
F-69006 Lyon (FR)**
Inventeur : **Perron, Robert
La Pecolière
F-69390 Charly (FR)**

(74) Mandataire : **Varniere-Grange, Monique et al
RHONE-POULENC RECHERCHES Centre de Recherches de Saint-Fons Service Brevets B.P. 62
F-69190 Saint-Fons (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

# Procédé de préparation d'anhydrides d'acides carboxyliques par carbonylation

La présente invention a pour objet un procédé de préparation d'anhydrides d'acides carboxyliques, et plus particulièrement de l'anhydride acétique, par carbonylation d'esters carboxyliques.

Il est bien connu que l'anhydride acétique peut être produit par carbonylation de l'acétate de méthyle, dans des conditions relativement sévères de pression en présence de complexes du nickel de formule

$$[A_4M]_2\,Ni\,X_4$$

dans laquelle X représente un atome de brome ou d'iode, M un atome de phosphore ou d'azote, A étant, par exemple, un radical alkyle inférieur. (Cf. brevet des Etats-Unis d'Amérique n° 2 729 651). Ces complexes, obtenus par réaction d'halogénures de nickel et d'halogénures de phosphonium ou d'ammonium quaternaire, peuvent être engagés sous cette forme dans la réaction en cause, ou bien ils peuvent être formés *in situ*. Toutefois, l'efficacité de ce type de procédé est faible malgré les hautes pressions mises en œuvre.

Plus récemment, il a été proposé des systèmes catalytiques permettant de carbonyler l'acétate de méthyle dans des conditions moins sévères de pression. Ainsi le brevet américain 4 002 678 décrit la carbonylation de l'acétate de méthyle en présence de nickel, de chrome, d'iodure de méthyle et d'une phosphine (ou d'une amine), sous une pression inférieure à 70 bars.

Parallèlement, il a été montré que le chrome n'est pas nécessaire dans un tel procédé lorsqu'on opère dans un acide carboxylique comme solvant (Cf. demande de brevet français 2 408 571). Néanmoins, le développement à l'échelle industrielle de ces techniques récentes, dont l'intérêt de principe n'est pas contesté, est compromis d'une part par l'instabilité et le coût des phosphines ou des amines nécessaires à leur mise en œuvre, et d'autre part, en raison de l'efficacité relativement faible des systèmes catalytiques en cause.

Il a maintenant été trouvé qu'il est possible de préparer des anhydrides d'acides carboxyliques, en particulier des anhydrides d'acides alcanoïques inférieurs et notamment l'anhydride acétique par carbonylation d'esters carboxyliques en présence de nickel, et d'au moins un promoteur iodé, avec une bonne productivité, dans des conditions de pression relativement douces, tout en obviant aux inconvénients précités.

La présente invention a donc pour objet un procédé perfectionné de carbonylation d'esters carboxyliques en phase liquide, en milieu sensiblement anhydre, en présence d'une quantité efficace de nickel, d'au moins un iodure d'alkyle ou d'acyle, d'une sulfone à titre de solvant et d'au moins un catalyseur choisi parmi les sels alcalins, les sels alcalino terreux, les iodures d'ammonium quaternaire et les iodures de phosphonium quaternaire.

Le procédé selon la présente invention peut être représenté par le schéma réactionnel ci-après :

$$R{-}CO{-}OR + CO \longrightarrow (RCO)_2O \qquad (1)$$

dans lequel R représente un radical alkyle ayant au maximum 12 atomes de carbone ou un radical $C_6H_5{-}C_xH_{2x}{-}$, x étant un entier compris entre 1 et 6, au maximum.

R est avantageusement un radical alkyle ayant de 1 à 4 atomes de carbone tel que les radicaux méthyle, éthyle, n-propyle, isopropyle, n-butyle, butyle secondaire ou tertiobutyle.

Par milieu sensiblement anhydre, la Demanderesse entend un milieu ne renfermant que des traces d'eau susceptibles de provenir des réactifs et/ou des composants du système catalytique lorsqu'on souhaite faire usage de produits disponibles dans le commerce.

Le procédé selon l'invention nécessite la présence d'une quantité efficace de nickel. N'importe quelle source de nickel peut être mise en œuvre dans le cadre du présent procédé. On peut introduire le nickel sous sa forme métallique (nickel RANEY, par exemple) ou sous toute autre forme commode. A titre d'exemples de composés du nickel susceptibles d'être utilisés pour la mise en œuvre du présent procédé, on peut citer : le carbonate, l'oxyde, l'hydroxyde, les halogénures, en particulier l'iodure, les carboxylates, en particulier l'acétate de nickel. Le nickel carbonyle convient également bien. On utilise de préférence le nickel RANEY, l'iodure de nickel, l'acétate de nickel et le nickel carbonyle.

La quantité de nickel n'est pas critique. La teneur en nickel qui a une influence sur la vitesse de réaction est déterminée en fonction de la vitesse de réaction que l'on estime convenable, compte tenu des autres paramètres de la réaction. De manière générale, une quantité comprise entre 5 et 2 000 milliatomes-grammes de nickel par litre de solution conduit à des résultats satisfaisants. On opère de préférence avec une teneur comprise entre 20 et 1 000 milliatomes-grammes de nickel par litre.

La mise en œuvre de la présente invention nécessite également la présence d'au moins un iodure d'alkyle ou d'acyle, c'est-à-dire d'au moins un composé de formule R'I (ou R'COI) dans laquelle R' a la signification donnée précédemment pour R, R' et R pouvant être identiques ou différents. On préfère utiliser les iodures d'alkyles ayant au maximum 4 atomes de carbone et plus particulièrement l'iodure de

méthyle ou d'éthyle.

Il n'est pas nécessaire de charger initialement ce type de composants du système catalytique. Bien entendu on peut charger initialement de l'iode libre ou de l'acide iodhydrique.

La Demanderesse a constaté que, dans le milieu réactionnel, les iodures alcalins (ou alcalinoterreux) peuvent être considérés comme des précurseurs d'iodures d'alkyles (ou d'acyles). Les iodures de lithium, de sodium et de potassium conviennent bien à la mise en œuvre du présent procédé. L'iodure de lithium s'avère particulièrement efficace.

En général la quantité d'iodure d'alkyle (ou d'acyle) ou de leur(s) précurseur(s) est telle que le rapport molaire I/Ni soit compris entre 1 et 100. Ce rapport est avantageusement fixé à une valeur comprise entre environ 3 et environ 50.

L'une des caractéristiques essentielles de la présente invention réside dans la mise en œuvre, à titre de solvant, d'une sulfone.

Les sulfones utilisables dans le cadre du présent procédé peuvent être représentées par la formule (I) ci-après :

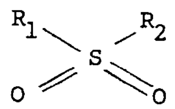

(I)

dans laquelle $R_1$ et $R_2$, identiques ou différents, représentent des radicaux alkyles ayant au maximum 4 atomes de carbone, $R_1$ et $R_2$ pouvant en outre former ensemble un radical unique divalent — alkylène ou alcénylène — comportant 3 à 6 atomes de carbone (par exemple, un radical tétraméthylène ou hexaméthylène) et le cas échéant 1 ou 2 doubles liaisons éthyléniques, ledit radical pouvant porter 1 à 3 substituants alkyles ayant de 1 à 4 atomes de carbone.

Une première classe de sulfones convenant à la mise en œuvre du présent procédé est constitué par les dialkylsulfones, c'est-à-dire par les sulfones de formule (I) ci-avant dans laquelle $R_1$ et $R_2$ sont identiques et représentent, de préférence, des radicaux alkyles linéaires ayant au maximum 4 atomes de carbone.

Une seconde catégorie de sulfones convenant particulièrement bien à la mise en œuvre du présent procédé est constituée par la tétraméthylènesulfone, la méthyl-3 tétraméthylènesulfone, la diméthyl-2,4 tétraméthylènesulfone et leurs mélanges.

En général la quantité de sulfone représente au moins 10 % en volume du milieu réactionnel ; de bons résultats sont obtenus lorsqu'on utilise de l'ordre de 20 % à 75 % (en volume) de sulfone.

Une seconde caractéristique essentielle du présent procédé réside dans la mise en œuvre d'au moins un cocatalyseur choisi parmi les sels alcalins, les sels alcalinoterreux, les iodures d'ammonium quaternaire et les iodures de phosphonium quaternaire.

Les sels alcalins et les sels alcalinoterreux utilisables dans le cadre du présent procédé répondent à la formule (II) ci-après :

$$M_b^{a+} X_c^{m-} \qquad (II)$$

dans laquelle a, m, b et c sont des entiers égaux à 1 ou à 2, dont les valeurs respectives sont choisies de sorte que la condition $a \times b = m \times c$ soit remplie et, lorsque a et m, sont identiques, b et c sont égaux à 1 ; M représente un atome de lithium, de sodium, de potassium, de césium, de rubidium, de calcium ou de magnésium et $X^{m-}$ est un anion choisi dans le groupe constitué par : $OH^-$, $Cl^-$, $Br^-$, $I^-$, $CO_3^=$, $NO_3^-$, $R''—O^-$ et $R''—CO—O^-$, R″ ayant la signification donnée précédemment pour R, R″ et R pouvant être identiques ou différents.

Parmi ces composés, les sels alcalins et notamment les sels de lithium, de sodium ou de potassium conviennent particulièrement bien à la mise en œuvre du présent procédé.

La nature précise de l'anion $X^{m-}$ ne paraît pas être un paramètre fondamental du présent procédé. A titre d'exemples de sels alcalins convenant à la mise en œuvre du présent procédé, on peut citer : LiOH, LiI, NaCl, KBr, NaI, KI, RbI, CsI, $NaNO_3$, $K_2CO_3$, $Li_2CO_3$, $CsNO_3$, l'acétate de lithium, de sodium ou de potassium, le méthylate de sodium ou de potassium et l'éthylate de sodium, de potassium ou de lithium. Les carboxylates alcalins (R″—COOM) et plus particulièrement les acétates sont d'un emploi commode et à ce titre peuvent être préconisés pour mettre en œuvre la présente invention.

La nature précise des iodures d'ammonium ou de phosphonium quaternaire, utilisables à titre de cocatalyseurs, n'est pas fondamentale dans le cadre du présent procédé. Le choix parmi ces composés est davantage orienté par des considérations d'ordre pratique, telles que la solubilité dans le milieu réactionnel, la disponibilité et la commodité d'emploi.

A ce titre, la Demanderesse préconise l'utilisation d'iodures d'ammonium ou de phosphonium quaternaire dont les cations sont représentés respectivement par les formules (III) et (IV) ci-après :

$$R_3N^+(R_4)_3 \qquad (III)$$

$$R_3P^+(R_4)_3 \qquad (IV)$$

dans lesquelles $R_3$ et $R_4$, identiques ou différents représentent des radicaux alkyles linéaires ayant au maximum 4 atomes de carbone, $R_4$ pouvant en outre représenter un radical phényle, tolyle ou xylyle.

A titre d'exemples d'iodures d'ammonium quaternaire, convenant à la mise en œuvre du présent procédé, on peut citer les iodures de tétraméthylammonium, triéthylméthylammonium, tributylméthylammonium, tributyl(n-propyl)ammonium, tétraéthylammonium et de tétrabutylammonium.

A titre d'exemples d'iodures de phosphonium quaternaire, convenant à la mise en œuvre du présent procédé, on peut citer les iodures de méthyltriphénylphosphonium, d'éthyltriphénylphosphonium, de méthyltrixylylphosphonium et de méthyltributylphosphonium.

On constatera à la lecture du présent mémoire que les iodures alcalins dans le procédé selon l'invention peuvent être considérés non seulement comme catalyseurs, mais encore comme précurseurs des iodures d'alkyles (ou d'acyles) dont il a été fait mention précédemment. En d'autres termes, lorsqu'on introduit un iodure alcalin dans le milieu réactionnel il n'est pas nécessaire d'ajouter un iodure d'alkyle (ou d'acyle) et/ou un des catalyseurs définis ci-avant.

Bien entendu, dans le cadre du présent procédé, il est possible d'utiliser plusieurs cocatalyseurs de l'une ou l'autre des catégories définies ci-avant. Ainsi, on peut mettre en œuvre un iodure alcalin et un carboxylate alcalin, par exemple l'iodure de sodium et l'acétate de lithium, ou l'iodure de lithium et l'acétate de potassium. De la même manière, on peut faire usage d'un iodure de phosphonium quaternaire et d'un carboxylate alcalin, par exemple de l'iodure de méthyltriphénylphosphonium et de l'acétate de lithium (ou de sodium).

En général la présence de 0,5 à 50 moles de cocatalyseur par atome-gramme de nickel conduit à des résultats satisfaisants. Pour une bonne mise en œuvre du procédé selon l'invention on utilise de 2 à 25 moles de cocatalyseur par atome-gramme de nickel.

Selon une variante du présent procédé, on peut ajouter au système catalytique défini ci-avant du chrome ou un composé du chrome.

Les composés du chrome utilisables dans le cadre de ce mode de réalisation, qui reste facultatif, sont plus particulièrement le chrome hexacarbonyle et les sels de formule (V) ci-après :

$$Cr_n^{D+} Y_q^{m-} \qquad (V)$$

dans laquelle q désigne le rapport (n × p)/m, p est égal à 2, 3, 4 ou 6, m ayant la signification précédente, n étant égal à 1 ou 2 et étant choisi en fonction des valeurs respectives de m et p de telle sorte que q soit entier, et Y a la signification donnée pour X dans la formule (II), Y pouvant en outre représenter un anion $O^=$, $HCOO^-$, $C_2O_4^=$ ou $CH_3COCHC(CH_3)O^-$.

Les carboxylates de chrome, notamment l'acétate de chrome (III), étant d'un emploi commode, peuvent être préconisés, à ce titre.

Lorsqu'on souhaite opérer dans le cadre de cette variante facultative, on utilise du chrome ou des composés du chrome dans des proportions par rapport au nickel sensiblement du même ordre que celles indiquées ci-avant pour le cocatalyseur, composant du système catalytique de base.

Selon la présente invention, on met en contact du monoxyde de carbone et un carboxylate d'alkyle en présence d'une sulfone à titre de solvant et du système catalytique défini précédemment.

La réaction est conduite en phase liquide sous une pression supérieure à la pression atmosphérique. En général, on opère sous une pression totale supérieure à 15 bars ; il n'est pas utile d'atteindre 700 bars. Pour une bonne mise en œuvre de l'invention, une pression totale de 25 à 200 bars est préconisée.

La température de réaction est généralement supérieure à 160 °C, sans qu'il soit nécessaire d'atteindre 300 °C. De bons résultats sont obtenus dans la gamme de températures allant de 180 à 220 °C.

On met en œuvre, de préférence, du monoxyde de carbone sous forme essentiellement pure, tel qu'il se présente dans le commerce. Toutefois, la présence d'impuretés telles que du dioxyde de carbone, de l'oxygène, du méthane et de l'azote peut être tolérée. La présence d'hydrogène n'est pas nuisible, même en des proportions relativement importantes.

En fin d'opération, l'anhydride carboxylique obtenu est séparé des autres constituants du milieu réactionnel par tout moyen approprié, par exemple par distillation.

Un avantage supplémentaire de la présente technique réside dans le fait que des compositions catalytiques particulièrement efficaces sont obtenues à partir d'espèces très accessibles et de structures extrêmement simples.

Une première catégorie de compositions catalytiques, dont la mise en œuvre constitue une variante préférée de la présente invention, est formée à partir de nickel et d'un iodure alcalin, en particulier un iodure de lithium, de sodium ou de potassium ; l'iodure de lithium s'averrant particulièrement efficace.

Une seconde catégorie de compositions catalytiques, dont l'utilisation constitue une autre variante avantageuse du présent procédé, est formée à partir de nickel, d'un iodure d'alkyle et d'un carboxylate alcalin. L'iodure d'alkyle est avantageusement l'iodure de méthyle ; le carboxylate alcalin est plus particulièrement un acétate, l'acétate de lithium convenant particulièrement bien.

Une dernière catégorie de compositions catalytiques préférées est obtenue à partir de nickel, d'un

iodure d'alkyle, d'un iodure alcalin et d'un carboxylate alcalin ; l'iodure de méthyle, l'iodure de sodium et l'acétate de lithium forment avec le nickel une composition catalytique particulièrement efficace.

Le procédé selon l'invention trouve une application particulièrement intéressante dans la préparation de l'anhydride acétique à partir de l'acétate de méthyle, dans la tétraméthylènesulfone.

Les exemples ci-après illustrent la présente invention sans toutefois en limiter le domaine ou l'esprit.

Les conventions suivantes sont utilisées ci-après :

— AcOme désigne l'acétate de méthyle
— TMS désigne la tétraméthylènesulfone
— $Ac_2O$ désigne l'anhydride acétique
— AcOH désigne l'acide acétique
— V désigne la vitesse initiale de la réaction exprimée en moles de monoxyde de carbone absorbées par heure.
— RR (%) désigne le nombre de moles d'anhydre acétique formées pour 100 moles d'acétate de méthyle chargées.
— Pr désigne la productivité en anhydride exprimée en grammes par heure et par litre de milieu réactionnel.

Exemple 1

Dans un autoclave de 125 ml de capacité en Hastelloy B-2, on charge :

— 25 ml (312 mMol) d'acétate de méthyle
— 20 ml de tétraméthylènesulfone
— 8 mAt-g de nickel sous forme d'acétate tétrahydraté
— 80 mMol d'iodure de méthyle et
— 20 mMol d'iodure de méthyltriphénylphosphonium.

Après fermeture de l'autoclave, on établit une pression de 40 bars de monoxyde de carbone. L'agitation par un système de va-et-vient est mise en route et l'autoclave est porté à 180 °C, en 25 minutes environ, au moyen d'un four annulaire. La pression dans l'autoclave est alors de 46 bars. Elle est ensuite maintenue constante et égale à 70 bars, par des recharges de monoxyde de carbone. La chute de pression de la réserve haute pression qui alimente en continu l'autoclave est enregistrée. Après deux heures de réaction à la température indiquée, l'agitation et le chauffage sont arrêtés ; l'autoclave est refroidi et dégazé. Le mélange réactionnel résultant est dilué et analysé par chromatographie gazeuse. Les résultats obtenus figurent dans le Tableau ci-après.

Essai témoin a

On reproduit l'exemple I en remplaçant la tétraméthylènesulfone par un volume identique d'acétate de méthyle.

Essai témoin b

On reproduit l'exemple I en remplaçant la tétraméthylènesulfone par le même volume d'acide acétique.

Les résultats des essais témoins a et b figurent également dans le tableau 1 ci-après qui montre clairement qu'en l'absence de solvant la réaction considérée n'a pas lieu, et que le remplacement de l'acide acétique par une sulfone permet d'augmenter considérablement la vitesse de carbonylation.

Tableau 1

| EX n° | AcOMe ml | Solvant Nature | Solvant ml | V | Ac2 O RR(%) | Ac2 O Pr |
|---|---|---|---|---|---|---|
| a | 45 | - | 0 | 0 | 0 | - |
| b | 25 | AcOH | 20 | 0,05 | 18 | 55 |
| 1 | 25 | T M S | 20 | 0,16 | 32 | 110 |

Exemple 2

On reproduit l'exemple 1 ci-avant, en remplaçant l'iodure de méthyltriphénylphosphonium par 40 mMol d'iodure de sodium.

Les résultats obtenus sont les suivants :

V = 0,12
RR = 28 %
Pr = 90 g/h × l

Exemple 3

On reproduit l'exemple 2 ci-avant en remplaçant l'iodure de sodium par une quantité équivalente d'acétate de lithium.

Les résultats obtenus sont les suivants :

V = 0,34
RR = 52 %
Pr = 160 g/h × l

Essai témoin c

On reproduit l'exemple 3 en remplaçant le solvant (TMS) par un volume équivalent d'acétate de méthyle ; il ne se produit aucune absorption du monoxyde de carbone.

Exemple 4

On reproduit l'exemple 2 ci-avant en ajoutant à la charge 4 mMol d'acétate de chrome (III).

Les résultats obtenus sont les suivants :

V = 0,27
RR = 58 %
Pr = 180 g/h × l

Exemple 5

On reproduit l'exemple 1 ci-avant, en ajoutant à la charge 4 mMol d'acétate de chrome III.

Les résultats obtenus sont les suivants :

V = 0,36
RR = 78 %
Pr = 250 g/h × l

Exemple 6

On reproduit l'exemple 2 ci-avant, en ajoutant à la charge 40 mMol d'acétate de lithium.

Les résultats obtenus sont les suivants :

V = 0,32
RR = 71 %
Pr = 230 g/h × l

Exemple 7

On reproduit l'exemple 1 ci-avant en ajoutant à la charge 40 mMol d'acétate de lithium.

Les résultats obtenus sont les suivants :

V = 0,35
RR = 65 %
Pr = 225 g/h × l

Essai témoin d

On reproduit l'essai témoin (a) en ajoutant à la charge 40 mMol d'acétate de lithium.

La vitesse (V) est égale à 0,05 et RR est inférieur à 5 %.

Exemple 8

Dans l'appareillage et selon le mode opératoire décrits ci-avant, on fait réagir du monoxyde de

carbone sur une charge constituée de 30 ml d'acétate de méthyle, 20 ml de tétraméthylènesulfone, 120 mMol d'iodure de sodium, 8 mAt-g de nickel sous forme d'acétate tétrahydraté, pendant deux heures, à 180 °C, sous une pression de 70 bars. En fin d'essai, on dose 2,6 g d'anhydride acétique. (RR = 7 %).

Exemple 9

Dans l'appareillage et selon le mode opératoire décrits ci-avant, on fait réagir du monoxyde de carbone sur une charge constituée de 23 ml d'acétate de méthyle, 20 ml de tétraméthylènesulfone, 110 mMol d'iodure de méthyle, 40 mMol d'acétate de potassium, 16 mMol d'acétate de nickel tétrahydraté, pendant 2 heures, sous une pression totale de 70 bars, la température n'étant que de 160 °C. En fin d'essai, on dose 1,3 g d'anhydride acétique (RR = 5 %). Cette quantité n'est pas négligeable compte tenu de la faible température de réaction choisie.

Exemple 10

On fait réagir un mélange de monoxyde de carbone et d'hydrogène dans le rapport molaire 2/1 sur une charge constituée de 26 ml d'acétate de méthyle, 20 ml de diméthyl-2,4 tétraméthylènesulfone, 8 mMol d'acétate de nickel tétrahydraté, 65 mMol d'iodure de méthyle, 50 mMol d'iodure de potassium. Après 2 heures de réaction, à 180 °C, sous une pression totale maintenue à 90 bars par des recharges de monoxyde de carbone, on dose 6,7 g d'anhydride acétique (RR = 21 %).

Essai témoin e

On reproduit l'exemple 10 ci-avant, en l'absence d'iodure de potassium et en utilisant 5 mMol d'iodure de nickel hexahydraté. Au bout de 3 heures 30 minutes d'essai, on n'observe aucune absorption du monoxyde de carbone.

Exemple 11

Dans l'appareillage et selon le mode opératoire décrits ci-avant, on fait réagir du monoxyde de carbone sur une charge constituée de 25 ml d'acétate de méthyle, 113 mMol d'iodure de méthyle, 60 mMol d'acétate de lithium, 20 mMol d'acétate de magnésium tétrahydraté, 10 mMol d'acétate de nickel tétrahydraté et 20 g de n-propylsulfone, pendant 2 heures à 180 °C sous une pression totale de 70 bars.

En fin d'essai, on dose 9,2 g d'anhydride acétique (RR = 29 %).

Exemple 12

Dans l'appareillage et selon le mode opératoire décrits ci-avant, on fait réagir du monoxyde de carbone sur une charge constituée de 25 ml d'acétate de méthyle, 81 mMol d'iodure de méthyle, 40 mMol d'acétate de lithium, 8 mMol d'acétate de nickel tétrahydraté et 20 ml de tétraméthylènesulfone, pendant 2 heures à 180 °C sous une pression totale de 40 bars.

En fin d'essai on dose : 11,8 g d'anhydride acétique (RR = 38 %).

Exemple 13

Dans l'appareillage et selon le mode opératoire décrits ci-avant, on fait réagir du monoxyde de carbone sur une charge constituée de 20 ml de tétraméthylènesulfone, 23 ml d'acétate de méthyle, 40 mMol d'acétate de potassium, 16 mMol d'acétate de nickel tétrahydraté et 100 mMol d'iodure de méthyle, pendant 2 heures, à 200 °C sous une pression totale de 70 bars.

En fin d'essai on dose 11,3 g d'anhydride acétique (RR = 39 %).

Exemple 14

Dans l'appareillage et selon le mode opératoire décrits ci-avant, on fait réagir du monoxyde de carbone sur une charge constituée de 30 ml d'acétate de méthyle, 20 ml de tétraméthylènesulfone, 8 mMol d'acétate de nickel tétrahydraté et 120 mMol d'iodure de lithium, pendant 2 heures à 180 °C sous une pression totale de 70 bars.

Les résultats obtenus sont les suivants :

V = 0,50
RR = 46 %
Pr = 170 g/h × l

Exemple 15

Dans l'appareillage et selon le mode opératoire décrits ci-avant, on fait réagir du monoxyde de carbone sur une charge constituée de 35 ml de tétraméthylènesulfone, 10 ml d'acétate de méthyle, 8 mMol d'acétate de nickel tétrahydraté, 40 mMol d'acétate de lithium, et 80 mMol d'iodure de méthyle, pendant 2 heures à 180 °C sous une pression totale de 70 bars.

Les résultats obtenus sont les suivants :

V = 0,27
RR = 60 %
Pr = 75 g/h × l

Exemple 16

Dans l'appareillage et selon le mode opératoire décrits ci-avant, on fait réagir du monoxyde de carbone sur une charge constituée de 24 ml d'acétate de méthyle, 20 ml de méthyl-3 tétraméthylènesulfone, 4 mMol d'acétate de nickel tétrahydraté, 97 mMol d'iodure de méthyle et 20 mMol de carbonate de lithium, pendant 2 heures à 180 °C sous une pression totale de 70 bars.

Les résultats obtenus sont les suivants :

V = 0,27
RR = 64 %
Pr = 190 g/h × l

Exemple 17

On reproduit l'exemple 3 mais sous une pression totale de 170 bars.

Les résultats obtenus sont les suivants :

V = 0,13
RR = 78 %
Pr = 245 g/h × l

Exemple 18

Dans l'appareillage et selon le mode opératoire décrits ci-avant, on fait réagir du monoxyde de carbone sur une charge constituée de 25 ml d'acétate de méthyle, 20 ml de tétraméthylènesulfone, 40 mMol d'acétate de calcium semi-hydraté, 80 mMol d'iodure de méthyle, 8 mMol d'acétate de nickel tétrahydraté et 40 mMol d'iodure de sodium, pendant 2 heures à 180 °C sous une pression totale de 70 bars.

En fin d'essai on dose 8,9 g d'anhydride acétique (RR = 29 %).

Exemple 19

Dans l'appareillage et selon le mode opératoire décrits ci-avant, on fait réagir du monoxyde de carbone sur une charge constituée de 35 ml d'acétate de méthyle, 10 ml de tétraméthylènesulfone, 40 mMol d'acétate de lithium, 8 mMol d'acétate de nickel tétrahydraté et 81 mMol d'iodure de méthyle, pendant 2 heures à 180 °C sous une pression totale de 70 bars.

Les résultats obtenus sont les suivants :

V = 0,14
RR = 38 %
Pr = 170 g/h × l

Exemple 20

Dans l'appareillage et selon le mode opératoire décrits ci-avant, on fait réagir du monoxyde de carbone sur une charge constituée de 23 ml d'acétate de méthyle, 20 ml de tétraméthylènesulfone, 16 mMol d'acétate de nickel tétrahydraté, 110 mMol d'iodure de méthyle et 40 mMol d'acétate de potassium pendant 2 heures à 200 °C sous une pression totale de 70 bars.

Les résultats obtenus sont les suivants :

V = 0,20
RR = 39 %
Pr = 115 g/h × l

Exemple 21

Dans l'appareillage et selon le mode opératoire décrits ci-avant, on fait réagir du monoxyde de carbone sur une charge constituée de 25 ml d'acétate de méthyle, 20 ml de tétraméthylènesulfone, 8 mMol d'acétate de nickel tétrahydraté, 20 mMol d'iodure de méthyltriphénylphosphonium, 80 mMol d'iodure de méthyle et 4 mMol de chrome hexacarbonyle, pendant 2 heures à 180 °C sous une pression totale de 70 bars.

Les résultats obtenus sont les suivants :

V = 0,27
RR = 43 %
Pr = 140 g/h × l

Exemple 22

On reproduit l'exemple 21 ci-avant, mais en remplaçant le chrome hexacarbonyle par 40 mMol d'acétate de chrome (III).

Les résultats obtenus sont les suivants :

V = 0,43
RR = 68,5 %
Pr = 220 g/h × l

Exemple 23

Dans l'appareillage et selon le mode opératoire décrits ci-avant, on fait réagir du monoxyde de carbone sur une charge constituée de 25 ml d'acétate de méthyle, 20 ml de tétraméthylènesulfone, 8 mMol de nickel tétracarbonyle, 80 mMol d'iodure de méthyle et 40 mMol d'acétate de lithium à 180 °C, sous une pression totale de 70 bars. L'absorption du monoxyde de carbone est terminée après 1 heure de réaction à 180 °C ; le chauffage est néanmoins poursuivi pendant encore 1 heure.

Les résultats obtenus sont les suivants :

V = 0,75
RR = 91 %
Pr = 575 g/h × l

**Revendications**

1. Procédé de préparation d'anhydrides d'acides carboxyliques par carbonylation d'esters carboxyliques de formule R—CO—OR dans laquelle R représente un radical alkyle ayant au maximum 12 atomes de carbone ou un radical $C_6H_5—C_xH_{2x}—$, x étant un entier compris entre 1 et 6 au maximum, en phase liquide, en milieu sensiblement anhydre caractérisé en ce que la réaction est conduite en présence d'une quantité efficace de nickel, d'au moins un iodure d'alkyle ou d'acyle, d'une sulfone à titre de solvant et d'au moins un cocatalyseur choisi parmi les sels alcalins, les sels alcalino terreux, les iodures d'ammonium quaternaire et les iodures de phosphonium quaternaire.

2. Procédé selon la revendication 1, caractérisé en ce que R est un radical alkyle ayant de 1 à 4 atomes de carbone.

3. Procédé selon la revendication 2, caractérisée en ce que R est un radical méthyle.

4. Procédé selon l'une quelconque des revendications précédentes, caractérisé en ce que la sulfone a pour formule

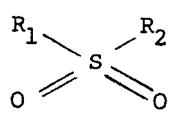

dans laquelle $R_1$ et $R_2$, identiques ou différents, représentent des radicaux alkyles ayant au maximum 4 atomes de carbone, $R_1$ et $R_2$ pouvant en outre former ensemble un radical unique divalent — alkylène ou alcénylène — comportant 3 à 6 atomes de carbone (par exemple, un radical tétraméthylène ou hexaméthylène) et le cas échéant 1 ou 2 doubles liaisons éthyléniques, ledit radical pouvant porter 1 à 3 substituants alkyles ayant de 1 à 4 atomes de carbone.

5. Procédé selon la revendication 4, caractérisé en ce que $R_1$ et $R_2$ sont identiques et représentent

des radicaux alkyles linéaires ayant au maximum 4 atomes de carbone.

6. Procédé selon la revendication 4, caractérisé en ce que la sulfone est choisie parmi la tétraméthylènesulfone, la méthyl-3 tétraméthylènesulfone, la diméthyl-2,4 tétraméthylènesulfone et leurs mélanges.

7. Procédé selon l'une quelconque des revendications précédentes, caractérisé en ce que la sulfone représente au moins 10 % en volume du milieu réactionnel.

8. Procédé selon la revendication 7, caractérisé en ce que la sulfone représente de 20 à 75 % en volume du milieu réactionnel.

9. Procédé selon l'une quelconque des revendications précédentes, caractérisé en ce que le cocatalyseur est chois parmi les iodures d'ammonium ou de phosphonium quaternaire respectivement de formules :

$$R_3N^+(R_4)_3 \qquad (III)$$

$$R_3P^+(R_4)_3 \qquad (IV)$$

dans lesquelles $R_3$ et $R_4$, identiques ou différents représentent des radicaux alkyles linéaires ayant au maximum 4 atomes de carbone, $R_4$ pouvant en outre représenter un radical phényle, tolyle ou xylyle.

10. Procédé selon l'une quelconque des revendications 1 à 8, caractérisé en ce que le cocatalyseur est un sel alcalin ou alcalino terreux de formule :

$$M_b^{a+} X_c^{m-}$$

dans laquelle a, m, b et c sont des entiers égaux à 1 ou à 2, dont les valeurs respectives sont choisies de sorte que la condition a × b = m × c soit remplie et, lorsque a et m sont identiques, b et c sont égaux à 1, M représente un atome de lithium, de sodium, de potassium, de césium, de rubidium, de calcium ou de magnésium et $X^{m-}$ est un anion choisi dans le groupe constitué par : $OH^-$, $Cl^-$, $Br^-$, $I^-$, $CO_3^=$, $NO_3^-$, R"—$O^-$ et R"—CO—$O^-$, R" ayant la signification donnée précédemment pour R, R" et R pouvant être identiques ou différents.

11. Procédé selon la revendication 10, caractérisé en ce que le cocatalyseur est un sel de lithium, de sodium ou de potassium.

12. Procédé selon la revendication 10 ou 11, caractérisé en ce que le cocatalyseur est un iodure.

13. Procédé selon la revendication 10 ou 11, caractérisé en ce que le cocatalyseur est un carboxylate.

14. Procédé selon la revendication 13, caractérisé en ce que le cocatalyseur est un acétate.

15. Procédé selon l'une quelconque des revendications précédentes, caractérisé en ce qu'on opère en présence d'un iodure d'alkyle ou d'acyle respectivement de formules R'I et R'COI dans lesquelles R' a la signification donnée précédemment pour R, R' et R pouvant être identiques ou différents.

16. Procédé selon la revendication 2 ou 3, caractérisé en ce qu'on opère en présence d'un iodure d'alkyle ayant au maximum 4 atomes de carbone, éventuellement formé *in situ* à partir d'un iodure alcalin ou alcalino terreux.

17. Procédé selon l'une quelconque des revendications précédentes, caractérisé en ce qu'on opère en présence de 5 à 2 000 et, de préférence de 20 à 1 000 milliatomes-grammes de nickel par litre de milieu réactionnel.

18. Procédé selon l'une quelconque des revendications précédentes, caractérisé en ce que le rapport molaire I/Ni est compris entre 1 et 100 et, de préférence entre 3 et 50.

19. Procédé selon l'une quelconque des revendications précédentes, caractérisé en ce que le rapport molaire du cocatalyseur au nickel est compris entre 0,5 et 50 et, de préférence entre 2 et 25.

20. Procédé selon l'une quelconque des revendications précédentes, caractérisé en ce que la température est supérieure à 160 °C et, est de préférence comprise entre 180 et 220 °C.

21. Procédé selon l'une quelconque des revendications précédentes, caractérisé en ce que la pression totale est comprise entre 15 et 700 bars et, de préférence entre 25 et 200 bars.

## Claims

1. Process for the preparation of carboxylic acid anhydrides by carbonylation of carboxylic esters of the formula R—CO—OR, in which R represents an alkyl radical having at most 12 carbon atoms or a radical $C_6H_5—C_xH_{2x}—$, in which x is an integer between 1 and at most 6, in the liquid phase in a substantially anhydrous medium, characterised in that the reaction is carried out in the presence of an effective amount of nickel, at least one alkyl or acyl iodide, a sulphone, as the solvent, and at least one cocatalyst chosen from amongst the alkali metal salts, the alkaline earth metal salts, quaternary ammonium iodides and quaternary phosphonium iodides.

2. Process according to Claim 1, characterised in that R is an alkyl radical having 1 to 4 carbon atoms.

3. Process according to Claim 2, characterised in that R is a methyl radical.

4. Process according to any of the preceding claims, characterised in that the sulphone has the formula

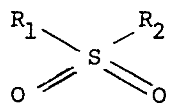

in which $R_1$ and $R_2$ are identical or different and represent alkyl radicals having at most 4 carbon atoms, or $R_1$ and $R_2$ together form a single divalent radical — alkylene or alkenylene — having 3 to 6 carbon atoms (for example a tetramethylene or hexamethylene radical) and optionally 1 or 2 ethylenic double bonds, it being possible for this radical to carry 1 to 3 alkyl substituents having 1 to 4 carbon atoms.

5. Process according to Claim 4, characterised in that $R_1$ and $R_2$ are identical and represent linear alkyl radicals having at most 4 carbon atoms.

6. Process according to Claim 4, characterised in that the sulphone is chosen from amongst tetramethylenesulphone, 3-methyl-tetramethylenesulphone, 2,4-dimethyltetramethylenesulphone and mixtures thereof.

7. Process according to any of the preceding claims, characterised in that the sulphone makes up at least 10 % by volume of the reaction mixture.

8. Process according to Claim 7, characterised in that the sulphone makes up 20 to 75 % by volume of the reaction mixture.

9. Process according to any of the preceding claims, characterised in that the cocatalyst is chosen from amongst ammonium iodides or quaternary phosphonium iodides which have, respectively, the formulae :

$$R_3N^+(R_4)_3 \qquad (III)$$

$$R_3P^+(R_4)_3 \qquad (IV)$$

in which $R_3$ and $R_4$ are identical or different and represent linear alkyl radicals having at most 4 carbon atoms, and $R_4$ can also represent a phenyl, tolyl, or xylyl radical.

10. Process according to any of Claims 1 to 8, characterised in that the cocatalyst is an alkali metal salt or alkaline earth metal salt of the formula :

$$M_b^{a+} X_c^{m-}$$

in which a, m, b and c are integers equal to 1 or 2, the respective values of which are chosen such that the condition $a \times b = m \times c$ is fulfilled and, if a and m are identical, b and c are equal to 1, M represents a lithium, sodium, potassium, cesium, rubidium, calcium or magnesium atom and $X^{m-}$ is an anion chosen from the group comprising $OH^-$, $Cl^-$, $Br^-$, $I^-$, $CO_3^=$, $NO_3^-$, $R''$—$O^-$ and $R''$—CO—$O^-$, in which R″ has the abovementioned meaning of R and R″ and R can be identical or different.

11. Process according to Claim 10, characterised in that the cocatalyst is a lithium, sodium or potassium salt.

12. Process according to Claim 10 or 11, characterised in that the cocatalyst is an iodide.

13. Process according to Claim 10 or 11, characterised in that the cocatalyst is a carboxylate.

14. Process according to Claim 13, characterised in that the cocatalyst is an acetate.

15. Process according to any of the preceding claims, characterised in that the reaction is carried out in the presence of an alkyl or acyl iodide of the respective formulae R′I and R′COI, in which R′ has the abovementioned meaning of R, and R′ and R can be identical or different.

16. Process according to Claim 2 or 3, characterised in that the reaction is carried out in the presence of an alkyl iodide which has at most 4 carbon atoms and may be formed *in situ* starting from an alkali metal iodide or alkaline earth metal iodide.

17. Process according to any of the preceding claims, characterised in that the reaction is carried out in the presence of 5 to 2,000, preferably 20 to 1,000, milligram atoms of nickel per litre of reaction mixture.

18. Process according to any of the preceding claims, characterised in that the molar ratio of I/Ni is between 1 : 1 and 100 : 1, preferably between 3 : 1 and 50 : 1.

19. Process according to any of the preceding claims, characterized in that the molar ratio of cocatalyst to nickel is between 0.5 : 1 and 50 : 1, preferably between 2 : 1 and 25 : 1.

20. Process according to any of the preceding claims, characterised in that the temperature is greater than 160 °C, and is preferably between 180 and 220 °C.

21. Process according to any of the preceding claims, characterised in that the overall pressure is between 15 and 700 bar, preferably between 25 and 200 bar.

**Ansprüche**

1. Verfahren zur Herstellung von Carbonsäureanhydriden durch Carbonylierung von Carbonsäureestern der Formel R—CO—OR, in der R eine Alkylgruppe mit maximal 12 Kohlenstoffatomen oder eine Gruppe $C_6H_5$—$C_xH_{2x}$— bedeutet, in der x eine ganze Zahl von 1 bis maximal 6 ist, in flüssiger Phase und in praktisch wasserfreiem Medium, dadurch gekennzeichnet, daß die Umsetzung in Gegenwart einer wirksamen Menge Nickel, mindestens eines Alkyl- oder Acyliodids, eines Sulfons als Lösungsmittel und mindestens eines Cokatalysators ausgewählt unter den Alkalisalzen, Erdalkalisalzen, quaternären Ammoniumiodiden und quaternären Phosphoniumiodiden, durchgeführt wird.

2. Verfahren nach Anspruch 1, dadurch gekennzeichnet, daß R eine Alkylgruppe mit 1 bis 4 Kohlenstoffatomen ist.

3. Verfahren nach Anspruch 2, dadurch gekennzeichnet, daß R eine Methylgruppe ist.

4. Verfahren nach einem der vorangegangenen Ansprüche, dadurch gekennzeichnet, daß das Sulfon der Formel

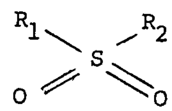

entspricht, in der $R_1$ und $R_2$ gleich oder verschieden sind und jeweils für Alkylgruppen mit maximal 4 Kohlenstoffatomen stehen, $R_1$ und $R_2$ weiterhin zusammen eine einzige zweiwertige Gruppe — Alkylen oder Aklenylen — bilden können, die 3 bis 6 Kohlenstoffatome enthält (beispielsweise die Tetramethylen- oder Hexamethylengruppe) und gegebenenfalls 1 oder 2 ethylenische Doppelbindungen, wobei diese Gruppe 1 bis 3 Alkylsubstituenten mit 1 bis 4 Kohlenstoffatomen tragen kann.

5. Verfahren nach Anspruch 4, dadurch gekennzeichnet, daß $R_1$ und $R_2$ identisch sind und für lineare Alkylgruppen mit maximal 4 Kohlenstoffatomen stehen.

6. Verfahren nach Anspruch 4, dadurch gekennzeichnet, daß das Sulfon ausgewählt wird unter Tetramethylensulfon, 3-Methyltetramethylensulfon, 2,4-Dimethyltetramethylensulfon und deren Gemischen.

7. Verfahren nach einem der vorangegangenen Ansprüche, dadurch gekennzeichnet, daß das Sulfon mindestens 10 Vol.-% des Reaktionsmediums ausmacht.

8. Verfahren nach Anspruch 7, dadurch gekennzeichnet, daß das Sulfon 20 bis 75 Vol.-% des Reaktionsmediums ausmacht.

9. Verfahren nach einem der vorangegangenen Ansprüche dadurch gekennzeichnet, daß der Cokatalysator ausgewählt wird unter den quaternären Ammonium- oder Phosphoniumiodiden der Formeln

$$R_3N^+(R_4)_3 \qquad (III)$$

$$R_3P^+(R_4)_3 \qquad (IV)$$

in denen $R_3$ und $R_4$ identisch oder verschieden voneinander sind und jeweils für lineare Alkylgruppen mit maximal 4 Kohlenstoffatomen stehen und $R_4$ weiterhin eine Phenyl-, Toluyl- oder Xylylgruppe sein kann.

10. Verfahren nach einem der Ansprüche 1 bis 8, dadurch gekennzeichnet, daß der Cokatalysator ein Alkali- oder Erdalkalisalz der Formel

$$M_b^a\ X_c^m$$

ist, in der a, m, b und c ganze Zahlen und zwar 1 oder 2 sind und ihr Zahlenwert so gewählt wird, daß die Bedingung a × b = m × c erfüllt wird und, wenn a und m identisch sind, b und c jeweils 1 bedeuten, M für ein Lithium-, Natrium-, Kalium-, Cäsium-, Rubidium-, Calcium- oder Magnesiumatom steht und $X^{m-}$ ein Anion aus der Gruppe $OH^-$, $Cl^-$, $Br^-$, $I^-$, $CO_3^-$, $NO_3^-$, R″—$O^-$ und R″—CO—$O^-$ ist, wobei R″ die oben für R gegebene Bedeutung hat und R″ und R gleich oder verschieden sein können.

11. Verfahren nach Anspruch 10, dadurch gekennzeichnet, daß der Cokatalysator ein Lithium-, Natrium- oder Kaliumsalz ist.

12. Verfahren nach Anspruch 10 oder 11, dadurch gekennzeichnet, daß der Cokatalysator ein Iodid ist.

13. Verfahren nach Anspruch 10 oder 11, dadurch gekennzeichnet, daß der Cokatalysator ein carbonsaures Salz ist.

14. Verfahren nach Anspruch 13, dadurch gekennzeichnet, daß der Cokatalysator ein Acetat ist.

15. Verfahren nach einem der vorangegangenen Ansprüche, dadurch gekennzeichnet, daß man in Gegenwart eines Alkyl- oder Acyliodids der Formel R′I bzw. R′COI arbeitet, in der R′ die zuvor für R gegebene Bedeutung hat und R′ und R gleich oder verschieden sein können.

16. Verfahren nach Anspruch 2 oder 3, dadurch gekennzeichnet, daß man in Gegenwart eines

Alkyliodids arbeitet, das maximal 4 Kohlenstoffatome enthält und gegebenenfalls ausgehend von einem Alkaliiodid oder Erdalkaliiodid *in situ* gebildet wird.

17. Verfahren nach einem der vorangegangenen Ansprüche, dadurch gekennzeichnet, daß man in Gegenwart von 5 bis 2 000 und vorzugsweise 20 bis 1 000 mg Atom Nickel je Liter Reaktionsmedium arbeitet.

18. Verfahren nach einem der vorangegangenen Ansprüche, dadurch gekennzeichnet, daß das Molverhältnis I/Ni 1 bis 100 und vorzugsweise 3 bis 50 beträgt.

19. Verfahren nach einem der vorangegangenen Ansprüche, dadurch gekennzeichnet, daß das Molverhältnis von Cokatalysator zu Nickel 0,5 bis 50 und vorzugsweise 2 bis 25 beträgt.

20. Verfahren nach einem der vorangegangenen Ansprüche, dadurch gekennzeichnet, daß die Temperatur über 160 °C, vorzugsweise im Bereich von 180 bis 220 °C liegt.

21. Verfahren nach einem der vorangegangenen Ansprüche, dadurch gekennzeichnet, daß der Gesamtdruck 15 bis 700 bar und vorzugsweise 25 bis 200 bar beträgt.